# EUROPÄISCHE PATENTANMELDUNG

(11) **EP 1 421 972 A2**
(43) Veröffentlichungstag der Anmeldung: **26.05.2004**
(21) Anmeldenummer: 03025902.2
(22) Anmeldetag: 12.11.2003
(51) Int. Cl.: A61N 1/05

(54) **Stimulationselektrode und deren Verwendung**

(30) Priorität: 20.11.2002 DE 10254287
(71) Anmelder: W. C. Heraeus GmbH & Co. KG, 63450 Hanau (DE)
(72) Erfinder: Frericks, Matthias, 63456 Hanau (DE); Specht, Heiko, 63739 Aschaffenburg (DE); Krüger, Frank, Dr., 63486 Bruchköbel (DE)
(74) Vertreter: Kühn, Hans-Christian

(57) **Zusammenfassung**

Die Erfindung betrifft eine Stimulationselektrode und deren Verwendung. Die Elektrodenoberfläche der Stimulationselektrode ist zumindest teilweise mit einer Beschichtung aus Titannitrid bedeckt, wobei das Titannitrid auf seiner der Elektrodenoberfläche abgewandten Seite eine höhere Oberfläche aufweist als der durch das Titannitrid bedeckte Bereich der Elektrodenoberfläche. Das Titannitrid ist erfindungsgemäß auf seiner der Elektrodenoberfläche abgewandten Seite mit mindestens einer Oxidationsschutzschicht bedeckt.

## Beschreibung

Die Erfindung betrifft eine Stimulationselektrode mit einer Elektrodenoberfläche, welche zumindest teilweise mit einer Beschichtung aus Titannitrid bedeckt ist, wobei das Titannitrid auf seiner der Elektrodenoberfläche abgewandten Seite eine höhere Oberfläche aufweist als der durch das Titannitrid bedeckte Bereich der Elektrodenoberfläche. Weiterhin betrifft die Erfindung die Verwendung einer solchen Stimulationselektrode.

Derartige Stimulationselektroden sind aus der DE 42 07 368 A1 bekannt. Die hier offenbarte Stimulationselektrode ist mit einer porösen Beschichtung versehen, wobei die Oberfläche der porösen Beschichtung größer ist, als die Oberflächengrundform der beschichteten Elektrode. Als Beschichtungsmaterialien sind dabei Nitride, Karbide, Karbonnitride bzw. reine Metalle oder Legierungen der Elemente Au, Ag, Ir, Pt sowie Kohlenstoff offenbart. Die offenbarte Stimulationselektrode wird als Herzschrittmacher- oder Neurostimulationselektrode eingesetzt.

Die US 2002/0002000 A1 offenbart Substrate aus Kunststoff, Metallen usw. mit einer biokompatiblen Beschichtung, die aus amorphen Titannitrid gebildet ist. Der Einsatz der Substrate erfolgt im Bereich von Herzschrittmachern und Elektroden.

Die EP 117 972 A, die EP 116 280 A sowie die EP 115 778 A offenbaren Elektroden für medizinische Anwendungen, die mit porösen Schichten aus Titannitrid versehen sind.

Die US 4,602,637 offenbart ein Herzschrittmachersystem, bei welchem die passive Elektrode z.B. mit aktiviertem Kohlenstoff oder Titannitrid beschichtet ist.

Die DE 33 00 672 A1 offenbart ein Herzschrittmachersystem mit einer Elektrode, welche beispielsweise eine Beschichtung aus Titannitrid aufweist.

Die Veröffentlichung "Improvement of Stimulation and Sensing Performance of Bipolar Pacemaker Leads", J. Riedmüller, A. Bolz, H. Rebling, M. Schaldach, Proceeding of the Annual International Conference of the IEEE/EMBS", (1992) 2364, offenbart, dass es beim Einsatz von Titannitridschichten für Stimulationselektroden mit anodischer Polung zu der Bildung von Oxidschichten im Bereich der Elektrodenfläche kommt. Während bei Stimulationselektroden mit einer Titannitridschicht, welche als Kathode eingesetzt werden, die physikalischen Eigenschaften des Titannitrids nicht wesentlich verändert werden, ist dies bei einem Betrieb als Anode nicht der Fall. Der Angriff durch OH⁻-lonen führt zu einer Bildung von Oxidschichten, die einen Anstieg der Impedanz und damit auch der Schwellenspannung verursachen. In Figur 2 der Veröffentlichung wird eine mit Iridium beschichtete Stimulationselektrode aus Titan einer gegenübergestellt, die mit Titannitrid beschichtet ist. Bei anodischem Betrieb sinkt die Helmholtzkapazität der mit Titannitrid beschichteten Elektrode im Hinblick auf die mit Iridium beschichtete Elektrode sehr viel schneller ab.

Es stellt sich nun das Problem, Stimulationselektroden mit zumindest teilweiser Titannitridbeschichtung bereitzustellen, die als Anode betrieben eine ausreichende Lebensdauer aufweisen.

Das Problem wird dadurch gelöst, dass das Titannitrid auf seiner der Elektrodenoberfläche abgewandten Seite mit mindestens einer Oxidationsschutzschicht bedeckt ist, wobei zumindest die der Beschichtung aus Titannitrid zugewandte Seite der Oxidationsschutzschicht im wesentlichen nicht porös ist. Die Oxidationsschutzschicht soll also zumindest auf ihrer der Beschichtung aus Titannitrid zugewandten Seite beziehungsweise an ihrer Grenzfläche zum Titannitrid dicht sein, jedoch ist es herstellungsbedingt fast unvermeidbar, dass Fehlstellen in der Oxidationsschutzschicht auftreten, die die Dichtigkeit zumindest punktuell beeinträchtigen können.
Die Verwendung einer solchen Oxidationsschutzschicht auf der Titannitridschicht führt dazu, dass die Bildung von Oxidschichten im Bereich der Stimulationselektrode vollständig unterbunden oder zumindest wesentlich verlangsamt wird.
Als Materialien für die Stimulationselektrode selbst sind Titan, Gold, Edelstahl, Platin, Platin-Iridium-Legierungen, insbesondere die Legierung Pt90Ir10, sowie Kohlenstoff bevorzugt.

Dabei hat es sich bewährt, dass die mindestens eine Oxidationsschutzschicht die Impedanz der mit Titannitrid beschichteten Stimulationselektrode senkt oder auf maximal einen Wert erhöht, der kleiner ist als die Impedanz der unbeschichteten Stimulationselektrode.

Außerdem hat es sich bewährt, wenn die mindestens eine Oxidationsschutzschicht eine Schichtdicke im Bereich von 100 nm bis 5 µm aufweist. Dabei ist zu beachten, dass eine ausreichende Oxidationsschutzwirkung erreicht wird sowie dass die Oberflächenstruktur des Titannitrids nicht wesentlich beeinflusst wird. So soll die Schichtdicke nach Möglichkeit so gewählt werden, dass die hohe Oberfläche des Titannitrids vollständig oder zumindest weitgehend erhalten bleibt. Insbesondere haben sich dabei Schichtdicken der mindestens einen Oxidationsschutzschicht im Bereich von 100 nm bis 2 µm, vorzugsweise im Bereich von 500 nm bis 2 µm, bewährt.

Besonders bevorzugt ist es dabei, wenn die Oxidationsschutzschicht biokompatibel ist.
Dabei ist es bevorzugt, wenn die mindestens eine Oxidationsschutzschicht aus mindestens einem der Elemente Iridium, Platin, Gold oder Kohlenstoff gebildet ist. Insbesondere haben sich hierbei reines Platin oder Iridium bewährt.
Weiterhin ist es bevorzugt, die mindestens eine Oxidationsschutzschicht aus einem Oxid, einem Karbid, einem Nitrid oder einem Polymer zu bilden, wobei allerdings zu beachten ist, dass Materialien verwendet werden, die die Impedanz der mit Titannitrid beschichteten Stimulationselektrode senken oder auf maximal einen Wert erhöhen, der kleiner ist als die Impedanz der unbeschichteten Stimulationselektrode.
Insbesondere hat sich dabei bewährt, Iridiumoxid, insbesondere unterstöchiometrisches Iridiumdioxid, als Oxidationsschutzschicht auf dem Titannitrid einzusetzen.

Vorzugsweise wird die mindestens eine Oxidationsschutzschicht durch ein PVD- (Physical Vapour Deposition) oder ein CVD- (Chemical Vapour Deposition) Verfahren gebildet. Aber auch die Bildung durch Aufsprühen, Tauchen, galvanische Abscheidung oder ein Sol-Gel-Verfahren ist möglich.

Die Verwendung einer solchen Stimulationselektrode mit einer Titannitridschicht und einer darauf angeordneten Oxidationsschutzschicht als Herzschrittmacherelektrode, Neurostimulationselektrode oder in einem anderen Human-Implantat ist ideal. Besonders vorteilhaft ist dabei der Einsatz der Stimulationselektrode als Anode.

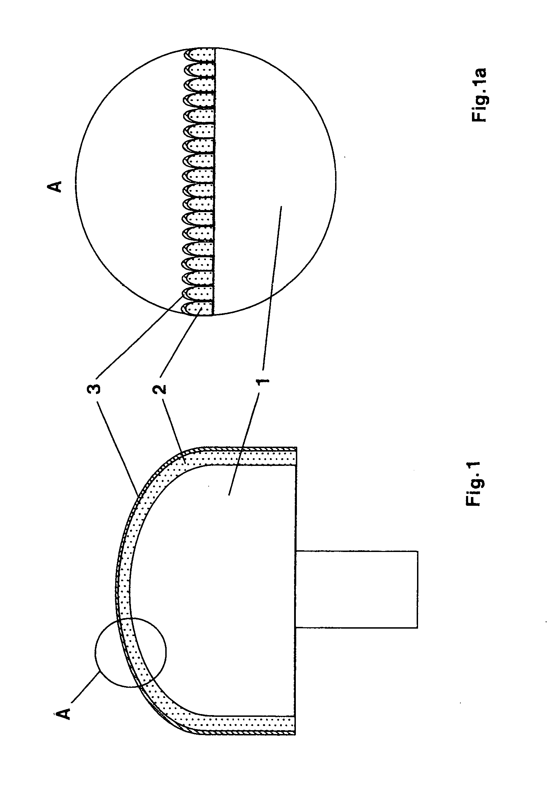

Die Figuren 1 und 1a sollen die erfindungsgemäße Stimulationselektrode beispielhaft erläutern. So zeigt:
- Figur 1: eine Stimulationselektrode mit Titannitridschicht und einer Oxidationsschutzschicht
- Figur 1a: den Ausschnitt A aus Figur 1 in vergrößerter Darstellung

Figur 1 zeigt die Stimulationselektrode 1. Die Form der Stimulationselektrode 1 kann auch jegliche andere Form annehmen als die hier dargestellte. So kann die Stimulationelektrode 1 beispielsweise auch in Form eines Spiraldrahtes ausgeführt sein. Die Elektrodenoberfläche der Stimulationselektrode 1 ist teilweise mit einer Beschichtung 2 aus Titannitrid bedeckt. Dabei ist die Beschichtung 2 aus Titannitrid auf ihrer der Elektrodenoberfläche der Stimulationselektrode 1 abgewandten Seite mit einer 1 µm dicken Oxidationsschutzschicht 3 aus Iridium bedeckt.

Figur 1a zeigt den Ausschnitt A aus Figur 1 im Bereich der Beschichtungen im Detail. Dabei ist zu erkennen, dass die Beschichtung 2 aus Titannitrid auf ihrer der Elektrodenoberfläche der Stimulationselektrode 1 abgewandten Seite eine höhere Oberfläche aufweist, als der durch das Titannitrid bedeckte Bereich der Elektrodenoberfläche. Die die Beschichtung 2 aus Titannitrid bedeckende Oxidationsschutzschicht 3 gibt die Oberflächenstruktur des Titannitrids weitgehend wieder, so dass die hohe Oberfläche der Beschichtung 2 aus Titannitrid ganz oder weitgehend erhalten bleibt.

## Patentansprüche

1. Stimulationselektrode mit einer Elektrodenoberfläche, welche zumindest teilweise mit einer Beschichtung aus Titannitrid bedeckt ist, wobei das Titannitrid auf seiner der Elektrodenoberfläche abgewandten Seite eine höhere Oberfläche aufweist als der durch das Titannitrid bedeckte Bereich der Elektrodenoberfläche, **dadurch gekennzeichnet, dass** das Titannitrid auf seiner der Elektrodenoberfläche abgewandten Seite mit mindestens einer Oxidationsschutzschicht bedeckt ist, wobei zumindest die der Beschichtung aus Titannitrid zugewandte Seite der Oxidationsschutzschicht nicht porös ist.

2. Stimulationselektrode nach Anspruch 1, **dadurch gekennzeichnet, dass** die mindestens eine Oxidationsschutzschicht die Impedanz der mit Titannitrid beschichteten Stimulationselektrode senkt oder auf maximal einen Wert erhöht, der kleiner ist als die Impedanz der unbeschichteten Stimulationselektrode.

3. Stimulationselektrode nach einem der Ansprüche 1 bis 2, **dadurch gekennzeichnet, dass** die mindestens eine Oxidationsschutzschicht eine Schichtdicke im Bereich von 100nm bis 5µm aufweist.

4. Stimulationselektrode nach Anspruch 3, **dadurch gekennzeichnet, dass** die Schichtdicke der mindestens einen Oxidationsschutzschicht 100nm bis 2µm beträgt.

5. Stimulationselektrode nach Anspruch 4, **dadurch gekennzeichnet, dass** die Schichtdicke der mindestens einen Oxidationsschutzschicht 500nm bis 2µm beträgt.

6. Stimulationselektrode nach einem der Ansprüche 1 bis 5, **dadurch gekennzeichnet, dass** die mindestens eine Oxidationsschutzschicht biokompatibel ist.

7. Stimulationselektrode nach einem der Ansprüche 1 bis 6, **dadurch gekennzeichnet, dass** die mindestens eine Oxidationsschutzschicht aus mindestens einem der Elemente Iridium, Platin, Gold oder Kohlenstoff gebildet ist.

8. Stimulationselektrode nach einem der Ansprüche 2 bis 6, **dadurch gekennzeichnet, dass** die mindestens eine Oxidationsschutzschicht aus einem Oxid, einem Karbid, einem Nitrid oder einem Polymer gebildet ist.

9. Stimulationselektrode nach Anspruch 8, **dadurch gekennzeichnet, dass** die mindestens eine Oxidationsschutzschicht aus Iridiumoxid gebildet ist.

10. Stimulationselektrode nach einem der Ansprüche 1 bis 9, **dadurch gekennzeichnet, dass** die mindestens eine Oxidationsschutzschicht durch ein PVD- oder ein CVD-Verfahren gebildet ist.

11. Stimulationselektrode nach einem der Ansprüche 1 bis 9, **dadurch gekennzeichnet, dass** die mindestens eine Oxidationsschutzschicht durch Aufsprühen, Tauchen, durch galvanische Abscheidung oder durch ein Sol-Gel-Verfahren gebildet ist.

12. Verwendung einer Stimulationselektrode nach einem der Ansprüche 1 bis 11 als Herzschrittmacherelektrode, Neurostimulationselektrode oder in einem anderen Human-Implantat..

13. Verwendung einer Stimulationselektrode nach Anspruch 12, **dadurch gekennzeichnet, dass** die Stimulationselektrode als Anode betrieben wird.
